# FASCICULE DE BREVET EUROPEEN

(11) **EP 1 458 338 B1**
(45) Date de publication et mention de la délivrance du brevet: **20.09.2006**
(21) Numéro de dépôt: 02805417.9
(22) Date de dépôt: 20.12.2002
(51) Int. Cl.: A61K 8/41, A61K 8/45, A61Q 5/10

(54) **COMPOSITION POUR LA TEINTURE D' OXYDATION DES FIBRES KERATINI QUES COMPRENANT UN AMIDE D ACIDE GRAS DE COLZA OXYETHYLENE**
FÄRBEZUSAMMENSETZUNG FÜR KERATINÖSE FASERN MIT EINEM OXYETHYLEN-RAPSSAMEN-FETTSÄUREAMID
DYEING COMPOSITION FOR KERATINOUS FIBERS COMPRISING AN OXYETHYLENE RAPESEED FATTY ACID AMIDE

(30) Priorité: 21.12.2001 FR 0116745
(43) Date de publication de la demande: 22.09.2004
(73) Titulaire: L'ORÉAL, 75008 Paris (FR)
(72) Inventeur: DESENNE, Patricia, F-92270 Bois Collombes (FR); BEBOT, Cécile, 92110 Clichy (FR); LAURENT, Florence, F-92270 Bois Colombes (FR)
(74) Mandataire: Wattremez, Catherine
(86) Numéro de dépôt international: PCT/FR2002/004519
(87) Numéro de publication internationale: WO 2003/053329

(56) Documents cités:
- EP-A- 0 166 100
- EP-A- 0 386 826

## Description

La présente invention concerne une composition pour la teinture d'oxydation des fibres kératiniques, en particulier des fibres kératiniques humaines et plus particulièrement les cheveux, comprenant, dans un milieu approprié pour la teinture au moins un colorant d'oxydation, et en outre au moins un amide d'acide gras de colza oxyéthyléné. L'invention concerne également les procédés et dispositifs de teinture mettant en oeuvre ladite composition.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para-phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur, elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Ces bases d'oxydation et ces coupleurs sont formulés dans des véhicules ou supports permettant leur application sur les fibres kératiniques après mélange avec un agent oxydant.
Ces véhicules sont généralement aqueux et pour que la composition de teinture ne coule pas sur le visage pendant le temps de pause, ils contiennent généralement un agent épaississant.
Ainsi pour obtenir des compositions de teinture d'oxydation faciles à appliquer sur la chevelure sans couler en dehors des zones que l'on se propose de teindre, on a utilisé par le passé des amides d'acides gras et notamment de coprah tels que le diéthanolamide d'acides de coprah ou le monoisopropanolamide d'acides de coprah, ou le monoéthanol-amide d'acide d'alkyl éther carboxylique oxyéthyléné par 2 moles d'oxyde d'éthylène.
EP-166 100 décrit des compositions de teinture d'oxidation contenant le diéthanolamide d'acide de coco.

Ces amides d'acides gras conduisent à des compositions de teinture d'oxydation avec de très bonnes qualités rhéologiques mais qui peuvent néanmoins être améliorées encore. EP-386 826 divulgue l'utilité de l'amide d'acide gras de colza oxyéthyléné pour des compositions contenant des tensioactifs.

C'est ainsi qu'après d'importantes recherches menées sur la question, il a été découvert de façon inattendue et tout à fait surprenante qu'il est possible d'obtenir des compositions de teinture d'oxydation dont les conditions d'application sont améliorées par rapport aux compositions de l'art antérieur en utilisant un amide d'acide gras de colza oxyéthyléné dans une certaine teneur.

Les compositions de teinture d'oxydation comprenant un amide d'acide gras de colza oxyéthyléné présentent donc un comportement rhéologique bien plus satisfaisant que celles de l'art antérieur avec d'autres amides d'acides gras (viscosité et/ou stabilité accrues), notamment après mélange avec la composition oxydante.

Elles permettent en outre d'obtenir des nuances puissantes et chromatiques avec de bonnes ténacités.

La présente invention a ainsi pour objet une composition pour la teinture d'oxydation des fibres kératiniques, en particulier des fibres kératiniques humaines, et plus particulièrement les cheveux, comprenant, dans un milieu approprié pour la teinture, au moins un colorant d'oxydation, et comprenant en outre plus de 5 % en poids de la composition, d'au moins un amide d'acide gras de colza oxyéthyléné.

Un autre objet de l'invention porté sur une composition prête à l'emploi pour la teinture d'oxydation des fibres kératiniques qui comprend, dans un milieu approprié pour la teinture, au moins une composition telle que décrite ci-avant et au moins un agent oxydant.
Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.

L'invention vise également un procédé de teinture d'oxydation des fibres kératiniques, et en particulier des fibres kératiniques humaines telles que les cheveux, consistant à appliquer sur les fibres une composition colorante comprenant, dans un milieu approprié pour la teinture, au moins un colorant d'oxydation et plus de 5 % en poids de la composition d'au moins un amide d'acide gras de colza oxyéthyléné, la couleur étant révélée à pH alcalin, neutre ou acide à l'aide d'une composition oxydante comprenant, dans un milieu approprié pour la teinture, au moins un agent oxydant, qui est mélangée juste au moment de l'emploi à la composition colorante ou qui est appliquée séquentiellement sans rinçage intermédiaire.

L'invention a également pour objet un dispositif de teinture à plusieurs compartiments ou "kit" pour la teinture d'oxydation des fibres kératiniques, en particulier des fibres kératiniques humaines et plus particulièrement les cheveux qui comporte un compartiment renfermant une composition colorante comprenant, dans un milieu approprié pour la teinture, au moins un colorant d'oxydation et plus de 5 % en poids de la composition d'au moins un amide d'acide gras de colza oxyéthyléné, et un autre compartiment renfermant une composition oxydante comprenant, dans un milieu approprié pour la teinture, un agent oxydant.

Mais d'autres caractéristiques, aspects, objets et avantages de l'invention apparaîtront encore plus clairement à la lecture de la description et des exemples qui suivent.

Les amides d'acide gras de colza oxyéthylénés utilisables selon la présente invention comportent plus particulièrement, en moyenne 1 à 20 moles d'oxyde d'éthylène, de préférence 1 à 10 moles d'oxyde d'éthylène et plus particulièrement 1 à 5 moles d'oxyde d'éthylène.
De manière avantageuse, l'amide correspond à la formule suivante : RCONH(CH₂CH₂O)ₙH, dans laquelle R dérive de l'acide gras issu de colza, n est un nombre moyen compris entre 1 et 20, de préférence entre 1 et 10, plus préférentiellement entre 1 et 5.
Parmi ces amides, on peut citer le produit commercial Aminol®N vendu par la société CHEM Y qui est un amide d'acide gras de colza comportant 4 moles d'oxyde d'éthylène.

Comme indiqué auparavant, le ou les amides d'acide gras de colza oxyéthylénés représentent plus de 5 % en poids de la composition.
Selon une variante plus particulière de l'invention, la teneur en amide d'acide gras de colza oxyéthyléné représente au moins 5,5 % en poids et de préférence au moins 6 % en poids de la composition.
Par ailleurs, la teneur en amide d'acide gras de colza oxyéthyléné représente jusqu'à 20 % en poids de la composition, et de préférence est inférieure à 10 % en poids de la composition. Il est à noter que la composition ne renferme pas l'oxydant.

### Colorants d'oxydation

Les colorants d'oxydation utilisables selon l'invention sont choisis parmi les bases d'oxydation et/ou les coupleurs.
De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :
- **(I)** les paraphénylènediamines de formule (I) suivante et leurs sels d'addition avec un acide : 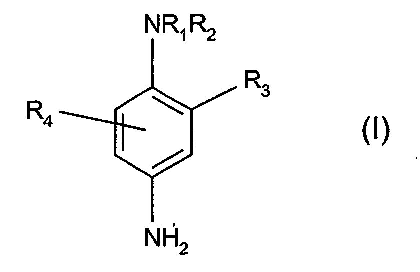 dans laquelle :
   R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

Parmi les groupements azotés de la formule (I) ci-dessus, on peut citer notamment les radicaux amino, monoalkyl( C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylène diamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropylparaphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)-amino 2-chloro-aniline, la 2-β-hydroxyéthylparaphénylènediamine, la 2-fluoro-paraphénylènediamine, la 2-isopropyl-paraphénylène diamine, la N-(β-hydroxypropyl)-paraphénylènediamine, la 2-hydroxyméthylparaphénylène-diamine, la N,N-diméthyl-3-méthyl-paraphénylène diamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylamino éthyloxy-paraphénylènediamine, la N-(β-méthoxyéthyl)-paraphénylènediamine, la 2-méthyl-1-N-β-hydroxyéthyl-paraphénylènediamine, la N-(4-aminophényl)-3-hydroxy-pyrrolidine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxy éthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- **(II)** Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 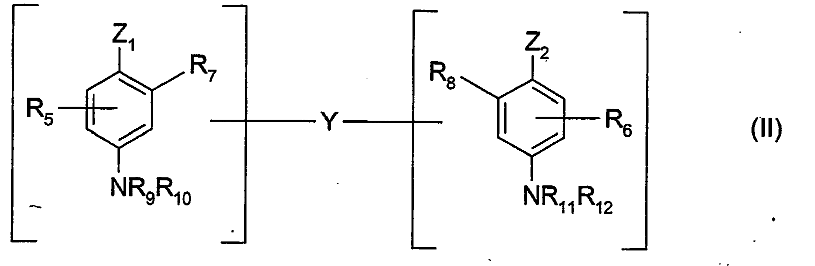 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- **(III)** les para-aminophénols répondant à la formule (III) suivante, et leurs sels d'addition avec un acide : 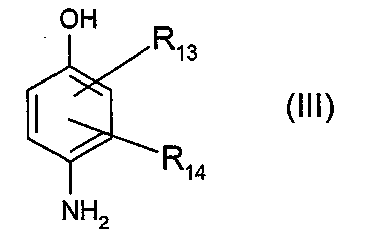 dans laquelle :
   **R**_{**13**} représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R**_{**14**} représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

- **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, la 2-(4-méthoxyphényl)amino-3-amino-pyridine, la 2,3-diamino-6-méthoxypyridine, la 2-(β-méthoxyéthyl)amino-3-amino-6-méthoxy pyridine, la 3,4-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétraaminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydrôxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition (ne comprenant pas l'oxydant) et encore plus préférentiellement de 0,005 à 8% en poids environ de ce poids.

Les coupleurs utilisables dans la composition de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-β-hydroxyéthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition (ne comprenant pas l'oxydant), et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition tinctoriale conforme à l'invention peut en outre comprendre un ou plusieurs colorants directs notamment pour modifier les nuances en les enrichissant de reflets. Ces colorants directs peuvent notamment être choisis parmi les colorants nitrés, azoïques ou anthraquinoniques, neutres, cationiques ou anioniques, classiquement utilisés ou ceux décrits notamment dans les demandes de brevet FR-2782450, 2782451, 2782452 et EP-1025834, dans la proportion pondérale d'environ 0,001 à 20% et de préférence de 0,01 à 10% du poids total de la composition.

La composition tinctoriale conforme à l'invention peut comprendre en outre au moins un agent tensioactif de nature anionique, non ionique, cationique ou amphotère dans une proportion allant de 0,01 à 40% et de préférence de 0,1 à 30% en poids par rapport au poids total de la composition.

Parmi les agents tensioactifs de type anionique, on préfère selon l'invention utiliser les tensioactifs faiblement anioniques dont on peut citer plus particulièrement parmi eux les acides éther carboxylique oxyalkylénés et leurs sels présentant la formule (I) suivante : 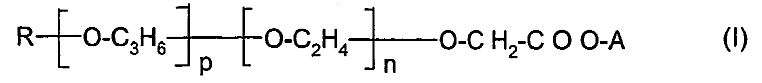 dans laquelle :
R représente un radical ou un mélange de radicaux alkyle ou alcényle linéaire ou ramifié en C₈-C₂₂, un radical alkyl(C₈-C₉)phényle, un radical R'CONH-CH₂-CH₂- avec R' désignant un radical alkyle ou alcényle linéaire ou ramifié en C₁₁-C₂₁,
n est un nombre entier ou décimal allant de 2 à 24,
p est un nombre entier ou décimal allant de 0 à 6,
A désigne un atome d'hydrogène ou bien Na, K, Li, 1/2Mg ou un reste monoéthanolamine, ammonium ou triéthanolamine.
Les acides éther carboxylique oxyalkylénés ou leurs sels utilisés de préférence selon la présente invention sont choisis parmi ceux de formule (I) dans laquelle R désigne un radical ou un mélange de radicaux alkyl(C12-C14), oléyle, cétyle, stéaryle ; un radical nonylphényle ou octylphényle, A désigne un atome d'hydrogène ou de sodium, p = 0, et n varie de 2 à 20 et de préférence 2 à 10.
Plus préférentiellement encore, on utilise des composés de formule (I) dans laquelle R désigne un radical alkyl(C₁₂), A désigne un atome d'hydrogène ou de sodium, p = 0, et n varie de 2 à 10.

Parmi les produits commerciaux, on peut utiliser de préférence les produits vendus par la société CHEM Y sous les dénominations :
AKYPO® NP 70 (R = nonylphényle, n = 7, p = 0, A = H) ;
AKYPO® NP 40 (R = nonylphényle, n = 4, p = 0, A = H) ;
AKYPO® OP 40 (R = octylphényle, n = 4, p = 0, A = H) ;
AKYPO® OP 80 (R = octylphényle, n = 8, p = 0, A = H) ;
AKYPO® OP 190 (R = octylphényle, n = 19, p = 0, A = H) ;
AKYPO® RLM 38 (R = alkyl(C12-C14), n = 3,8, p = 0, A = H) ;
AKYPO® RLM 38 NV (R = alkyl(C12-C14), n = 4, p = 0, A = Na) ;
AKYPO® RLM 45 (R = alkyl(C12-C14), n = 4,5, p = 0, A = H) ;
AKYPO® RLM 45 NV (R = alkyl(C12-C14), n = 4,5, p = 0, A = Na) ;
AKYPO® RLM 100 (R = alkyl(C12-C14), n = 10, p = 0, A = H) ;
AKYPO® RLM 100 NV (R = alkyl(C12-C14), n = 10, p = 0, A = Na) ;
AKYPO® RLM 130 (R = alkyl(C12-C14), n = 13, p = 0, A = H) ;
AKYPO® RLM 160 NV (R = alkyl(C12-C14), n = 16, p = 0, A = Na) ;
AKYPO® RO 20 (R = oléyle, n = 2, p = 0, A = H) ;
AKYPO® RO 90 (R = oléyle, n = 9, p = 0, A = H) ;
AKYPO® RCS 60 (R = cétyle/stéaryle, n = 6, p = 0, A = H) ;
AKYPO® RS 60 (R = stéaryle, n = 6, p = 0, A = H) ;
AKYPO® RS 100 (R = stéaryle, n = 10, p = 0, A = H) ;
AKYPO® RO 50 (R = oléyle, n = 5, p = 0, A = H),
ou par la société SANDOZ sous les dénominations :
SANDOPAN ACA-48 (R = cétyle/stéaryle, n = 24, p = 0, A = H) ;
SANDOPAN DTC-Acid (R = alkyl(C₁₃), n = 6, p = 0, A = H) ;
SANDOPAN DTC (R= alkyl(C₁₃), n = 6, p = 0, A = Na) ;
SANDOPAN LS 24 (R = alkyl(C₁₂-C₁₄), n = 12, p = 0, A = Na) ;
SANDOPAN JA 36 (R= alkyl(C₁₃), n = 18, p = 0, A = H),
et plus particulièrement, les produits vendus sous les dénominations suivantes : AKYPO® NP 70 ; AKYPO® NP 40 ; AKYPO® OP 40; AKYPO® OP 80 ; AKYPO® RLM 25 ; AKYPO® RLM 45 ; AKYPO® RLM 100 ; AKYPO® RO 20 ; AKYPO® RO 50 ; AKYPO® RLM 38.

Les acides éther carboxyliques oxyalkylénés ou leurs sels représentent environ de 2 à 15%, et de préférence environ de 3 à 10% du poids total de la composition.

Parmi les agents tensioactifs non ioniques qui sont des composés bien connus en soi (voir notamment à cet égard "Handbook of Surfactants" par M.R. PORTER, éditions Blackie & Son (Glasgow and London), 1991, pp 116-178), on peut citer (liste non limitative) les alcools, les alpha-diols, les alkylphénols polyéthoxylés ou polypropoxylés, ayant une chaîne grasse comportant par exemple 8 à 22 atomes de carbone, le nombre de groupements oxyde d'éthylène ou oxyde de propylène pouvant aller notamment de 1 à 50. On peut également citer les copolymères d'oxyde d'éthylène et de propylène, les condensats d'oxyde d'éthylène et de propylène sur des alcools gras ; les alcools gras mono- ou poly-glycérolés comportant en moyenne 1 à 30 groupements glycérol ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

Selon l'invention, on préfère utiliser :
1) les alcools gras comportant de 8 à 22 atomes de carbone et oxyéthylénés par 1 à 10 moles d'oxyde d'éthylène. Parmi eux, on peut citer plus particulièrement l'alcool laurique 2 OE, l'alcool laurique 3 OE, l'alcool décylique 3 OE et l'alcool décylique 5 OE.
2) les alcools gras mono- ou poly-glycérolés que l'on peut représenter par la formule (II) suivante :
   dans laquelle : 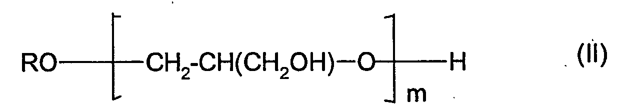
   R représente un radical saturé ou insaturé, linéaire ou ramifié, comportant de 8 à 40 atomes de carbone et de préférence de 10 à 30 atomes de carbone ;
   m représente un nombre allant de 1 à 30 et de préférence de 1 à 10.

Comme composés de ce type, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol , l'alcool oléocétylique à 6 moles de glycérol, et l'octadécanol à 6 moles de glycérol.

L'alcool gras peut représenter un mélange d'alcools gras au même titre que la valeur de m représente une valeur statistique, ce qui signifie que dans un produit commercial peuvent coexister plusieurs espèces d'alcools gras polyglycérolés sous forme d'un mélange.
Parmi les alcools gras mono- ou poly-glycérolés, on préfère plus particulièrement utiliser l'alcool en C8/C10 à 1 mole de glycérol, l'alcool en C10/C12 à 1 mole de glycérol et l'alcool en C12 à 1,5 mole de glycérol.

Le ou les tensioactifs non ioniques représentent environ de 2 à 40% en poids, et de préférence environ de 4 à 20% du poids total de la composition.

La composition prête à l'emploi selon l'invention peut également comprendre dans la composition colorante et/ou la composition oxydante des polymères cationiques ou amphotères tels que décrits ci-dessous et d'autres agents d'ajustement de la rhéologie tels que les épaississants cellulosiques (hydroxyéthycellulose, hydroxypropylcellulose, carboxyméthylcellulose..), la gomme de guar et ses dérivés (hydroxypropylguar..), les gommes d'origine microbienne (gomme de xanthane, gomme de scléroglucane..), les homopolymères réticulés d'acide acrylique ou d'acide acrylamidopropanesulfonique et les polymères associatifs tels que décrits ci-dessous.
Préférentiellement selon l'invention, la partie colorante comprend au moins un polymère cationique ou amphotère et au moins un polymère associatif.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP 337 354 et dans les brevets français FR 2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus. Ils sont notamment décrits dans les brevets FR 2 505 348 ou FR 2 542 997. Parmi lesdits polymères, on peut citer :
**(1)** Les homopolymères ou copolymères dérivés d'esters ou d'amides acryliques ou méthacryliques et comportant au moins un des motifs de formules (V), (VI), (VII) ou (VIII) suivantes: 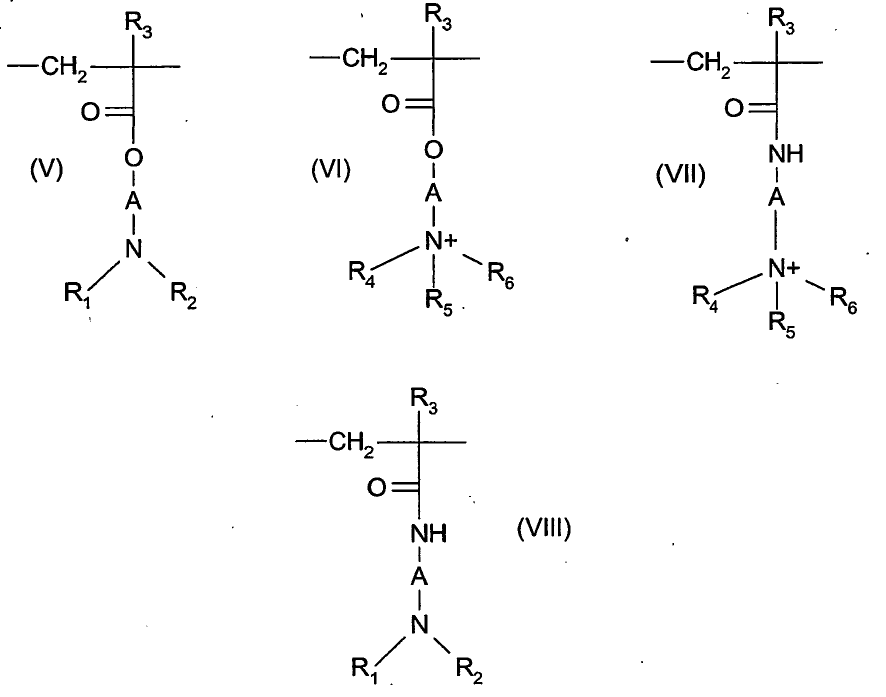 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate
   ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
- les copolymères d'acrylamide et de diméthylaminoéthyl méthacrylate quaternisé au sulfate de diméthyle ou avec un hologénure de diméthyle, tel que celui vendu sous la dénomination HERCOFLOC® par la société HERCULES,
- les copolymères d'acrylamide et de chlorure de méthacryloyloxyéthyltriméthyl-ammonium décrits par exemple dans la demande de brevet EP-A-080976 et vendus sous la dénomination BINA QUAT P 100® par la société CIBA GEIGY,
- le copolymère d'acrylamide et de méthosulfate de méthacryloyloxyéthyltriméthyl-ammonium vendu sous la dénomination RETEN® par la société HERCULES,
- les copolymères vinylpyrrolidone / acrylate ou méthacrylate de dialkylaminoalkyle quaternisés ou non, tels que les produits vendus sous la dénomination "GAFQUAT®" par la société ISP comme par exemple "GAFQUAT® 734" ou "GAFQUAT® 755" ou bien les produits dénommés "COPOLYMER® 845, 958 et 937". Ces polymères sont décrits en détail dans les brevets FR 2.077.143 et FR 2.393.573,
- les terpolymères méthacrylate de diméthyl amino éthyle/ vinylcaprolactame/vinylpyrrolidone tel que le produit vendu sous la dénomination GAFFIX® VC 713 par la société ISP,
- les copolymère vinylpyrrolidone / méthacrylamidopropyl dimethylamine commercialisés notamment sous la dénomination STYLEZE® CC 10 par ISP,
- et les copolymères vinylpyrrolidone / méthacrylamide de diméthyl-amino-propyle quaternisés tel que le produit vendu sous la dénomination "GAFQUAT® HS 100" par la société ISP.

**(2)** Les dérivés d'éthers de cellulose comportant des groupements ammonium quaternaire décrits dans le brevet français 1 492 597, et en particulier les polymères commercialisés sous les dénominations "JR®" (JR 400, JR 125, JR 30M) ou "LR®" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec' un sel de méthacryloyléthyl triméthylammonium, de méthacrylamido propyl triméthylammonium, de diméthyl-diallylammonium. Les produits commercialisés répondant à cette définition sont plus particulièrement les produits vendus sous la dénomination "Celquat® L 200" et "Celquat® H 100" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR® C13 S, JAGUAR® C 15, JAGUAR® C 17 ou JAGUAR® C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quatemisées. De tels polymères sont notamment décrits dans les brevets FR 2.252.840 et FR 2.368.508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet FR 1.583.363.
   Parmi ces dérivés, on peut citer plus particulièrement les polymères acide adipique/diméthylaminohydroxypropyl/diéthylène triamine vendus sous la dénomination "Cartaretine ®F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets US 3.227.615 et US 2.961.347.
   Des polymères de ce type sont en particulier commercialisés sous la dénomination "Hercosett® 57" par la société Hercules Inc. ou bien sous la dénomination de "PD 170®" ou "Delsette® 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (IX) ou (X) : 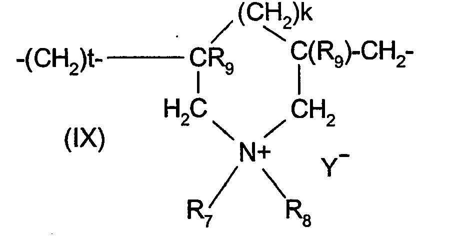 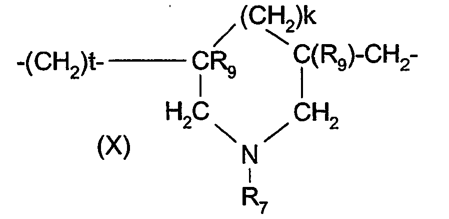 formules dans lesquelles k et t sont égaux à 0 ou 1, la somme k + t étant égale à 1 ; Rg désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet FR 2.080.759 et dans son certificat d'addition n°2.190.406.
   Parmi les polymères définis ci-dessus, on peut citer plus particulièrement l'homopolymère de chlorure de diméthyldiallylammonium vendu sous la dénomination "Merquat® 100" par la société Calgon (et ses homologues de faible masse moléculaire moyenne en poids) et les copolymères de chlorure de diallyldiméthylammonium et d'acrylamide commercialisés sous la dénomination "MERQUAT® 550".
**(10)** Le polymère de diammonium quaternaire contenant des motifs récurrents répondant à la formule : 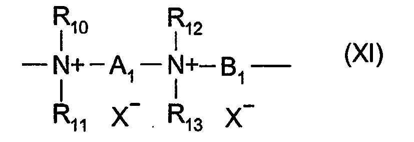 formule (XI) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
      De préférence, X⁻ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (XII) suivante: 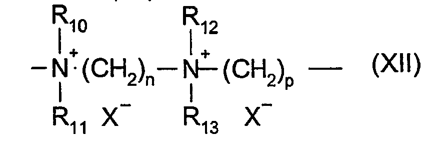 dans laquelle R₁₀, R₁₁, R₁₂ et R₁₃, identiques ou différents, désignent un radical alkyle
ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs récurrents de formule (XIII) : 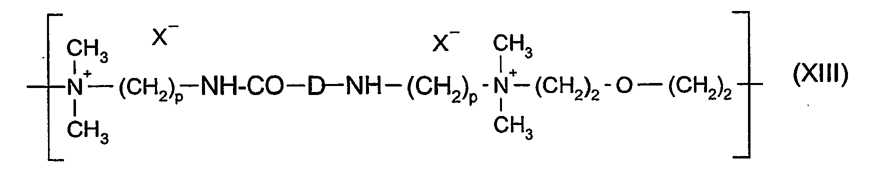 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets US 4157 388, US 4 702 906, US 4 719 282. Ils sont notamment décrits dans la demande de brevet EP 122 324. Parmi eux, on peut par exemple citer, les produits "Mirapol® A 15", "Mirapol® AD1", "Mirapol® AZ1" et "Mirapol® 175" vendus par la société Miranol.
**(12)** Les polymères quaternaires de vinylpyrrolidone et de vinylimidazole tels que par exemple les produits commercialisés sous les dénominations Luviquat® FC 905, FC 550 et FC 370 par la société B.A.S.F.
**(13)** Les polyamines comme le Polyquart® H vendu par HENKEL, référencé sous le nom de "POLYETHYLENEGLYCOL (15) TALLOW POLYAMINE " dans le dictionnaire CTFA.
(14) Les polymères réticulés de sels de méthacryloyloxyalkyt(C₁-C₄) trialkyl(C₁₋C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92" par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10), (11), (12) et (14) et encore plus préférentiellement les polymères aux motifs récurrents de formules (W) et (U) suivantes : 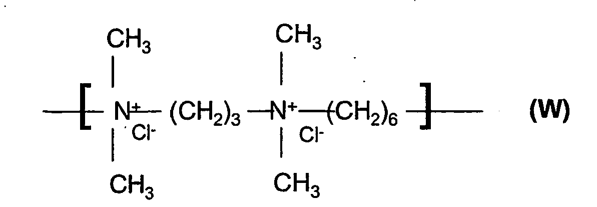 et notamment ceux dont le poids moléculaire, déterminé par chromatographie par perméation de gel, est compris entre 9500 et 9900; 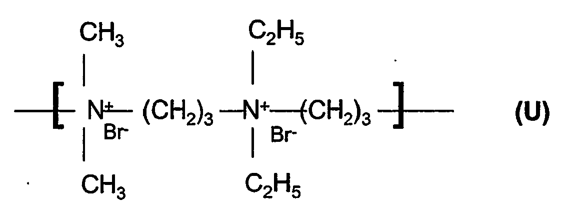 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.
Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkyl-amino-alkyl-méthacrylate et acrylate, les dialkyl-amino-alkyl-méthacrylamide et acrylamide. De tels composés sont décrits dans le brevet US 3 836 537. On peut également citer le copolymère acrylate de sodium / chlorure d'acrylamidopropyl trimethyl ammonium vendu sous la dénomination POLYQUART® KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT® 280, MERQUAT® 295 et MERQUAT® PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiobctyl-acrylamide, le N-octylacrylamide, le N-décylacrylamide, le N-dodécylacrylamide ainsi que les méthacrylamides correspondants. Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER® ou LOVOCRYL® 47 par la société NATIONAL STARCH.
(3) Les polyaminoamides réticulés et alcoylés partiellement ou totalement dérivant de polyaminoamides de formule générale : 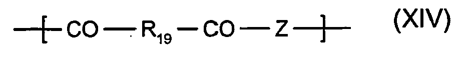 dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 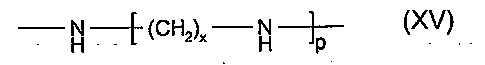 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XV) ci-dessus, dans lequel x=2 et p=1 et qui dérive de l'éthylènediamine, ou le radical dérivant de la pipérazine : 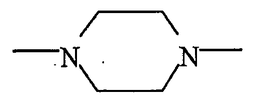
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 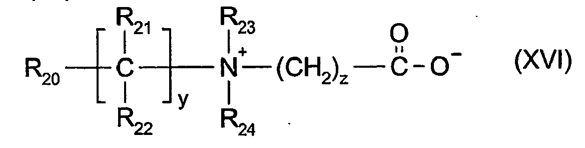 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthyl-aminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl-carboxy-méthyl-ammonio-éthyl-méthacrylate de méthyle tel que le produit vendu sous la dénomination DIAFORMER® Z301 par la société SANDOZ.
(5) les polymères dérivés du chitosane comportant des motifs monomères répondant aux formules (XVII), (XVIII), (XIX) suivantes: 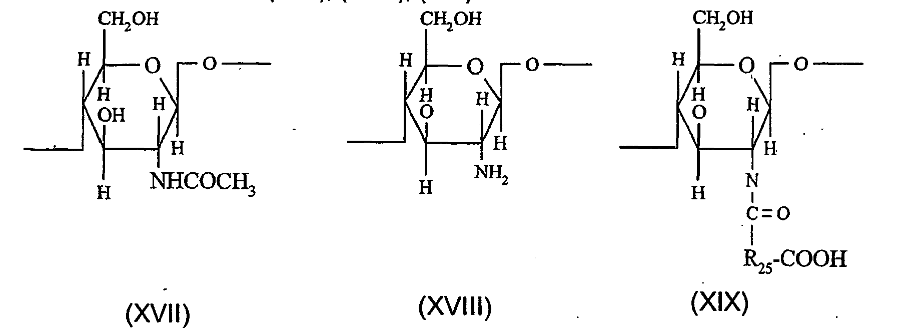 le motif (XVII) étant présent dans des proportions comprises entre 0 et 30%, le motif (XVIII) dans des proportions comprises entre 5 et 50% et le motif (XIX) dans des proportions comprises entre 30 et 90%, étant entendu que dans ce motif (XIX), R₂₅ représente un radical de formule : 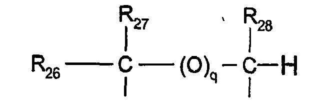 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN®" par la société JAN DEKKER.
(7) Les polymères répondant à la formule générale (XX) tels que ceux décrits par exemple dans le brevet français 1 400 366 : 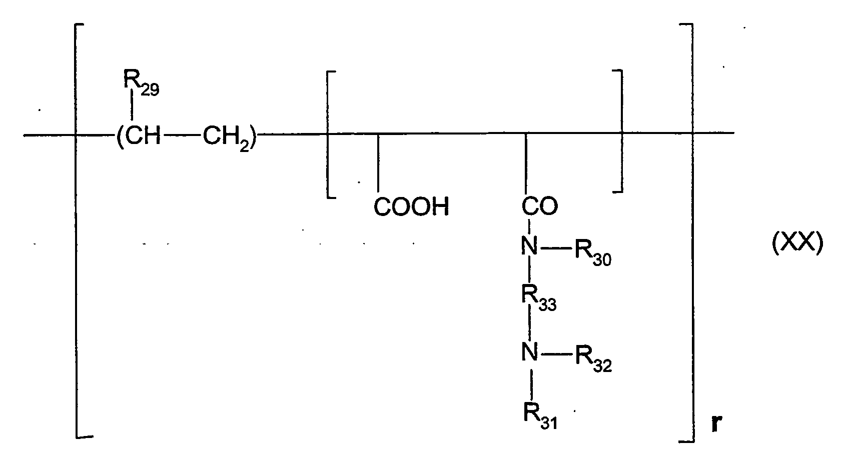 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus, ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XXI)**

      où D désigne un radical 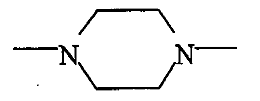 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XXII)**

      où D désigne un radical 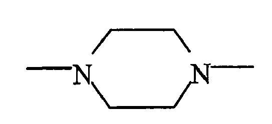 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)-vinyléther / anhydride maléique modifié partiellement par semiamidification avec une N,N-dialkylaminoalkylamine telle que la N,N-diméthyl-amino-propylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

### Polymères associatifs utilisables selon l'invention

Les polymères associatifs sont des polymères hydrosolubles capables, dans un milieu aqueux, de s'associer réversiblement entre eux ou avec d'autres molécules.
Leur structure chimique comprend des zones hydrophiles, et des zones hydrophobes caractérisées par au moins une chaîne grasse.
Les polymères associatifs selon l'invention peuvent être de type anionique, cationique, amphotère et de préférence non ionique ou cationique.

### Polymères associatifs de type anionique :

On peut citer parmi eux :
- **(I)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = C R' CH₂ O Bₙ R (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80® et SALCARE SC90® qui sont des émulsions aqueuses à 30% d'un terpolymère réticulé d'acide méthacrylique, d'acrylate d'éthyle et de steareth-10-allyl éther (40/50/10).
- **(II)** ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 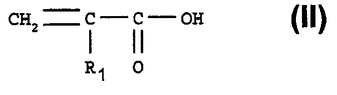
dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante 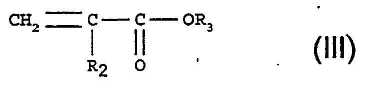 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂. Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US 3 915 921 et US 4 509 949.
Parmi ce type de polymères associatifs anioniques, on utilisera plus particulièrement des polymères formés à partir d'un mélange de monomères comprenant :
(i) essentiellement de l'acide acrylique,
(ii) un ester de formule (III) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précedemment.
   Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

- **(III)** les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- **(IV)** les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.
   Préférentiellement ces composés comprennent également comme monomère un ester d'acide carboxylique à insaturation α,β-monoéthylénique et d'alcool en C1C4.
   A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

### Polymères associatifs de type cationique

On peut citer parmi eux :
- **(I)** les polyuréthanes associatifs cationiques dont la famille a été décrite par la demanderesse dans la demande de brevet français N°-0009609; elle peut être représentée par la formule générale (la).suivante :

   R-X-(P)ₙ-[L-(Y)ₘ]ᵣ-L'-(P')ₚ-X'-R' (la)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25,
   n, m, et p valent chacun indépendamment des autres entre 0 et 1000 ;
   la molécule contenant au moins une fonction amine protonée ou quaternisée et au moins un groupement hydrophobe.

Dans un mode de réalisation préféré de ces s polyuréthanes, les seuls groupements hydrophobes sont les groupes R et R' aux extrémités de chaîne.

Une famille préférée de polyuréthanes associatifs cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (la) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (la) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 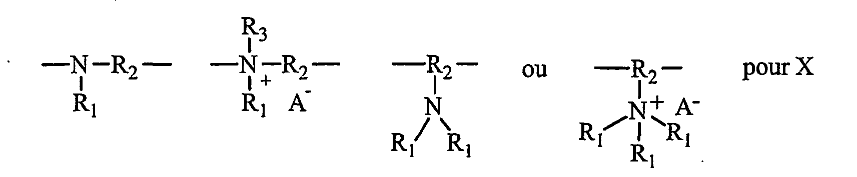 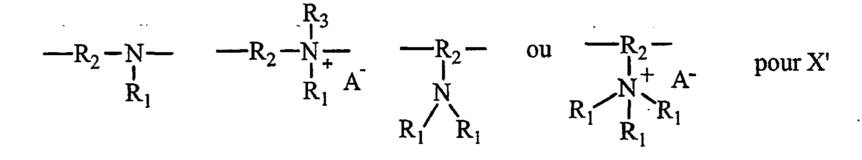 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 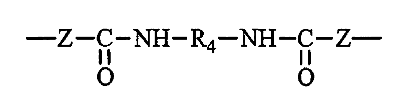 dans laquelle:
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 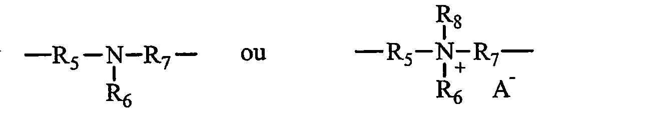 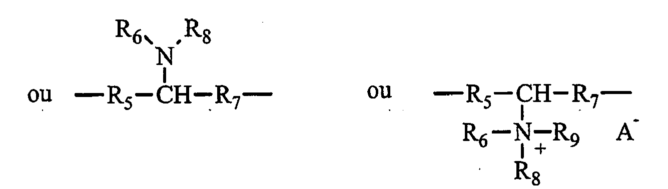 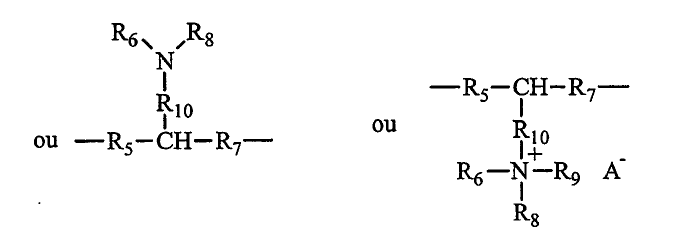 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P, et A⁻ est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (Ia) selon l'invention sont formés à partir de diisocyanates et de différents composés possédant des fonctions à hydrogène labile. Les fonctions à hydrogène labile peuvent être des fonctions alcool, amine primaire
ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (la) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine.
Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (la) est un diisocyanate correspondant à la formule :

O=C=N-R₄-N=C=O

dans laquelle R₄ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (la) est un composé hydrophobe destiné à former les groupes hydrophobes terminaux du polymère de formule (la).
Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné □-hydroxyle. Le groupe hydrophobe du polyuréthane de formule (la) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire,
ou thiol. Ce composé peut être un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (la) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.
- **(II)** les dérivés de cellulose quaternisée et les polyacrylates à groupements latéraux aminés non cycliques.
   Les dérivés de cellulose quaternisée sont en particulier,
   - les celluloses quatemisées modifiées par des groupements comportant au moins une chaîne grasse, tels que les groupes alkyle, arylalkyle, alkylaryle comportant au moins 8 atomes de carbone, ou des mélanges de ceux-ci,
   - les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.
   Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle. On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.
- **(III)** Les polyvinyllactames cationiques dont la famille a été décrite par la demanderesse dans la demande de brevet français N°-0101106.
   Lesdits polymères comprennent :
   - a) au moins un monomère de type vinyl lactame ou alkylvinyllactame;
   - b) au moins un monomère de structures (I) ou (II) suivantes : 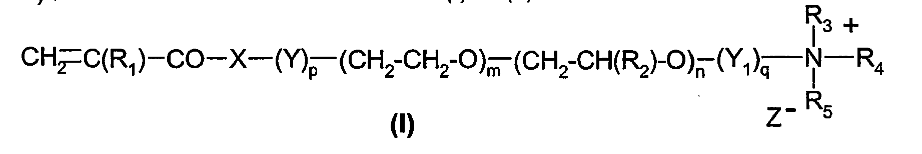 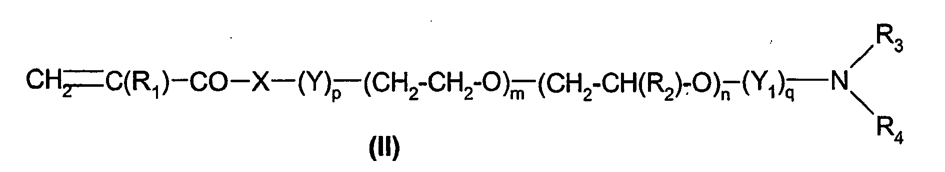
   dans lesquelles :
   X désigne un atome d'oxygène ou un radical NR₆,
   R₁ et R₆ désignent, indépendamment l'un de l'autre, un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent, indépendamment l'un de l'autre, un atome d'hydrogène, un radical alkyle linéaire ou ramifié en C₁-C₃₀ ou un radical de formule (III) :

      ―(Y₂)ᵣ― (CH₂-CH(R₇)-O)ₓ―R₈ (III)
   Y, Y₁ et Y₂ désignent, indépendamment l'un de l'autre, un radical alkylène linéaire ou ramifié en C₂-C₁₆,
   R₇ désigne un atome d'hydrogène, ou un radical alkyle linéaire ou ramifié en C₁-C₄ ou un radical hydroxyalkyle linéaire ou ramifié en C₁-C₄,
   R₈ désigne un atome d'hydrogène ou un radical alkyle linéaire ou ramifié en C₁-C₃₀,
   p, q et r désignent, indépendamment l'un de l'autre, soit la valeur zéro, soit la valeur 1,
   m et n désignent, indépendamment l'un de l'autre, un nombre entier allant de 0 à 100,
   x désigne un nombre entier allant de 1 à 100,
   Z désigne un anion d'acide organique ou minéral,
   sous réserve que :
   - l'un au moins des substituants R₃, R₄, R₅ ou R₈ désigne un radical alkyle linéaire ou ramifié en C₉-C₃₀,
   - si m ou n est différent de zéro, alors q est égal à 1,
   - si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence le contre ion Z⁻ des monomères de formule (I) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.
Plus préférentiellement, le monomère b) est un monomère de formule (I) pour laquelle, encore plus préférentiellement, m et n sont égaux à zéro.

Le monomère vinyl lactame ou alkylvinyllactame est de préférence un composé de structure (IV) : 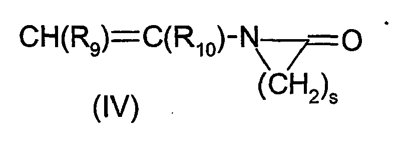 dans laquelle :
s désigne un nombre entier allant de 3 à 6,
Rg désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (IV) est la vinylpyrrolidone.

Les polymères poly(vinyllactame) cationiques selon l'invention peuvent également contenir un ou plusieurs monomères supplémentaires, de préférence cationiques ou non ioniques.

A titre, de composés plus particulièrement préférés selon l'invention, on peut citer les terpolymères suivants comprenant au moins :
a)-un monomère de formule (IV),
b)-un monomère de formule (I) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (II) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b).
De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide/ tosylate de cocoyldiméthylméthacrylamido propylammonium, les terpolymères vinylpyrrolidone / diméthylaminopropyl méthacrylamide / tosylate ou chlorure de lauryldiméthylméthacrylamidopropylammonium..

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

### Polymères associatifs amphotères

Ils sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (Ia) ou (Ib): 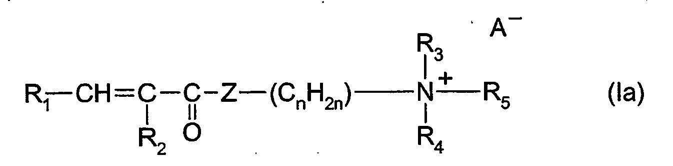 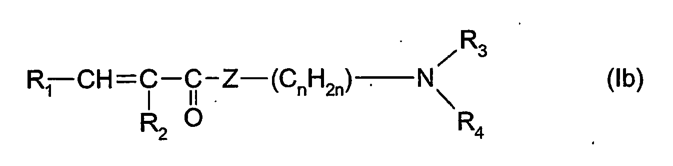
   dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (II)

   R₆-CH= CR₇-COOH **(II)**

   dans laquelle, R₆ et R₇, identiques ou différents, représentent un atome d'hydrogène ou un radical méthyle; et
3) au moins un monomère de formule (III) :

   R₆-CH= CR₇- COXR₈ **(III)**

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ; l'un au moins des monomères de formule (la), (Ib) ou (III) comportant au moins une chaîne grasse.

Les monomères de formule (la) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (la) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 moles% du monomère comportant une chaîne grasse (monomère de formule (la), (Ib) ou (III)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.
Parmi les polymères associatifs amphotères selon l'invention, on préfère les terpolymères acide acrylique/chlorure de (méth)acrylamidopropyl triméthyl ammonium/ méthacrylate de stéaryle.

### Polymères associatifs de type non ionique

Selon l'invention, ils sont choisis de préférence parmi :
- (1) les celluloses modifiées par des groupements comportant au moins une chaîne grasse ;
   on peut citer à titre d'exemple:
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHODIA.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.
On peut également citer le produit ELFACOS T210® à chaîne alkyle en C₁₂₋₁₄ et le produit ELFACOS T212® à chaîne alkyle en C₁₈ de chez AKZO.
Le produit DW 1206B® de chez ROHM & HAAS à chaîne alkyle en C₂₀ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).
Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
De tels polyéther polyuréthanes sont vendus notamment par la société ROHM & HAAS sous les appellations Aculyn 46® et Aculyn 44® [l'ACULYN 46® est un polycondensat de polyéthylène glycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Les polymères cationiques ainsi que les polymères amphotères peuvent représenter environ de 0,01 % à 10 % en poids, de préférence de 0,05 % à 5 % en poids, et encore plus préférentiellement de 0,1 % à 3 % en poids, du poids total de la composition.
Les polymères associatifs peuvent représenter environ de 0,01 à 10% du poids total de la composition et de préférence de 0,1 à 5% en poids du poids total de la composition.

### Milieu

Le milieu de la composition colorante approprié pour la teinture, est de préférence un milieu aqueux constitué par de l'eau pouvant comprendre avantageusement un ou plusieurs solvants organiques acceptables sur le plan cosmétique, dont plus particulièrement, des monoalcools ou des diols, linéaire ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyl(C1-C4)éthers de diéthylèneglycol comme par exemple, le monoéthyléther ou le monobutyléther du diéthylèneglycol, seuls
ou en mélange.

Les solvants peuvent alors être présents, chacun, dans des concentrations comprises entre 0,5 et 20% en poids par rapport au poids total de la composition. Plus particulièrement, la teneur en solvant est d'au moins 2 % en poids, de préférence d'au moins 5 % en poids par rapport au poids total de la composition. Par ailleurs, elle est de préférence inférieure ou égale à 20 % en poids, avantageusement inférieure ou égale à 15 % en poids par rapport au poids total de la composition.

### Autres adjuvants

La composition colorante selon l'invention peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Selon une caractéristique de l'invention, la composition peut comprendre au moins un corps gras, choisi notamment parmi les alcools gras saturés, linéaires ou ramifiés, comprenant au moins 10 atomes de carbone ; les huiles ou cires, saturées, d'origine végétale, hydrocarbonées. Avantageusement, si de tels composés sont présents, leur teneur totale est inférieure ou égale à 10 % en poids de la composition.

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

La composition tinctoriale conforme à l'invention peut en outre comprendre des alcools gras insaturés tels que par exemple l'alcool oléique, dans une proportion en poids allant de 0,5 à 15% par rapport au poids total de la composition de teinture.

Bien entendu, l'homme de l'art veillera à choisir le ou les éventuels composés complémentaires mentionnés ci-avant, de manière telle que les propriétés avantageuses attachées intrinsèquement à la composition prête à l'emploi selon l'invention ne soient pas, ou substantiellement pas, altérées par la ou les adjonctions envisagées.

Conformément à un mode de réalisation particulier de l'invention, la composition, avant le mélange avec la composition oxydante, se présente sous la forme d'un liquide à 25°C et à pression atmosphérique.
De préférence, ledit liquide présente une viscosité d'au plus 150 cPs, mesurée avec le rhéomètre RhéoStress 1, taux de cisaillement de 200s⁻¹, à 25°C.

### Oxydant

Dans la composition oxydante, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement compris entre les valeurs 3 et 12, bornes incluses. Il est de préférence compris entre 8,5 et 11 bornes incluses, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante : 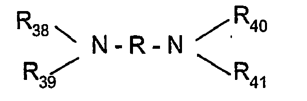 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en Ci-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions colorante et oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Un exemple concret illustrant l'invention est indiqué ci-après, sans pour autant présenter un caractère limitatif.

### EXEMPLE

On a préparé la composition de teinture suivante :

### Composition colorante :

### (exprimée en grammes de Matière active)

| | |
|---|---|
| Acide lauryl éther carboxylique 4,5 OE (Akypo® RLM 45 vendu par CHEM Y) | 7 |
| Alcool laurique 2 OE (Dehydol®LS-2-DEO-N vendu par COGNIS) | 4 |
| Alcool décylique 5OE (Empilan®KA-5/90-FL vendu par ALBRIGHT & WILSON) | 8 |
| Alcool oléique | 3 |
| Amide d'acides gras de colza à 4 moles d'oxyde d'éthylène (Aminol®N vendu par CHEM Y) | 7 |
| Polymère associatif non ionique (Dapral® T212 vendu par AKZO) | 1 |
| Monoéthanolamine | 2 |
| Polyquaternium 6 (Merquat® 100 vendu par CALGON) | 1,5 |
| Ethanol | 11 |
| Propylène glycol | 5 |
| Dipropylène glycol | 5 |
| 1,3-dihydroxybenzène (résorcinol) | 0,3 |
| Paraphénylènediamine | 0,3 |
| Réducteurs, antioxydants | q.s. |
| Séquestrant | q.s. |
| Parfum | q.s. |
| Ammoniaque (à 20,5% en ammoniac) | 1,6 |
| Eau déminéralisée qsp | 100 |

La composition colorante a été mélangée, au moment de l'emploi, dans un bol en plastique et pendant 2 minutes, à une composition oxydante titrant 20 volumes en eau oxygénée à raison de 1 partie de composition colorante pour 1,5 parties de composition oxydante.
On a appliqué le mélange obtenu sur des mèches de cheveux à 90% de blancs et on a laissé poser 30 minutes.
On a ensuite rincé les mèches à l'eau, on les a lavées au shampooing, à nouveau rincées à l'eau, puis séchées et démêlées.
On a obtenu alors une nuance brun verdâtre.

### EXEMPLE 2

On a préparé les compositions de teinture suivantes :

### Compositions colorantes :

**(teneurs exprimées en grammes de matière active)**

| | Composition A Invention | Composition B Comparatif |
|---|---|---|
| Propylène glycol | 5 | 5 |
| Amide d'acides de colza oxyéthyléné (4 OE) | 7 | 3 |
| Acide lauryl éther carboxylique (4,5 OE) | 4,5 | 4,5 |
| Alcool décylique oxyéthyléné (3 OE) | 8,0 | 8,0 |
| Alcool oléique | 1 | 1 |
| Alcool laurylique oxyéthyléné (2 OE) | 4 | 4 |
| Diuréthane (HMDI) d'alcools (C16/C18) oxyéthylénés (66 OE) et oxypropylénés (14 OP) | 0,1 | 0,1 |
| 1,3-dihydroxybenzène (résorcinol) | 0,3 | 0,3 |
| 1,4-diamino-benzène | 0,3 | 0,3 |
| Monoéthanolamine pure | 2 | 2 |
| Acide éthylène diamine tétracétique | 0,2 | 0,2 |
| Acide érythorbique (ou acide D-isoascorbique) | 0,31 | 0,31 |
| Homopolymère chlorure de diméthyl diallyl ammonium en solution aqueuse à 40 % | 1,5 | 1,5 |
| Parfum | 0,95 | 0,95 |
| Dipropylène glycol | 5 | 5 |
| Thiolactate d'ammonium en solution aqueuse à 58 % (50 % en acide thiolactique) | 0,8 0,8 | 0,8 0,8 |
| Ammoniaque (concentration de référence à 20%) | 8 | 8 |
| Alcool éthylique 96 degrés dénaturé | 11,0 | 11,0 |
| Eau désionisée | Qsp 100 | Qsp 100 |

La composition colorante a été mélangée, au moment de l'emploi, à une composition oxydante titrant 20 volumes en eau oxygénée à raison de 1 partie de composition colorante pour 1,5 parties de composition oxydante.
Le mélange obtenu est appliqué par demi-tête sur 10 modèles différents (une demi-tête avec la composition A mélangée à la composition oxydante et l'autre demi-tête avec la composition B mélangée à la même composition oxydante).

Les différences d'application entre les 2 supports sont alors notées sur une échelle de 1 à 5 et en particulier :
- l'adhérence en racine (1 : peu adhérent - 5 : très adhérent)
- la consistance sur tête (1 : peu épais - 5 : très épais)

Sur une moyenne de 10 essais, l'évaluation ayant été réalisée par un panel d'expert, (l'évaluateur changeant à chaque essai), on obtient les résultats suivants :

| Adhérence | | Consistance | |
|---|---|---|---|
| A | B | A | B |
| 3,35 | 2,25 | 2,95 | 1,95 |

La composition A selon l'invention, qui contient 7% d'Aminol N, est bien plus adhérente en racines et plus consistante sur tête (écarts significatifs) que la composition B comparative qui ne contient que 3% d'Aminol N.

## Revendications

1. Composition pour la teinture d'oxydation pour fibres kératiniques, en particulier pour fibres kératiniques humaines et plus particulièrement des cheveux, comprenant dans un milieu approprié pour la teinture, au moins un colorant d'oxydation, **caractérisée par le fait qu'**elle comprend plus de 5 % en poids de la composition d'au moins un amide d'acide gras de colza oxyéthyléné.

2. Composition selon la revendication 1, **caractérisée par le fait que** l'amide d'acide gras de colza oxyéthyléné est choisi dans le groupe formé par ceux comportant 1 à 20 moles d'oxyde d'éthylène.

3. Composition selon la revendication 2, **caractérisée par le fait que** l'amide d'acide gras de colza oxyéthyléné est choisi dans le groupe formé par ceux comportant 1 à 10 moles d'oxyde d'éthylène.

4. Composition selon la revendication 3, **caractérisée par le fait que** l'amide d'acide gras de colza oxyéthyléné est choisi dans le groupe formé par ceux comportant 1 à 5 moles d'oxyde d'éthylène.

5. Composition selon l'une quelconque des revendications précédentes, **caractérisée par le fait que** la teneur en amide d'acide gras de colza oxyéthyléné représente jusqu'à 20 % en poids de la composition, de préférence inférieure à 10 % en poids de la composition.

6. Composition selon l'une des revendications précédentes, **caractérisé par le fait que** la teneur en amide représente au moins 5,5 % en poids, de préférence au moins 6 % en poids de la composition.

7. Composition selon l'une quelconque des revendications précédentes, **caractérisée par le fait que** le colorant d'oxydation est choisi parmi les bases d'oxydation et les coupleurs.

8. Composition selon la revendication 7, **caractérisée par le fait qu'**elle comprend au moins une base d'oxydation.

9. Composition selon les revendications 7 ou 8, **caractérisée par le fait que** les bases d'oxydation sont choisies parmi les ortho- ou para- phénylènediamines, les bases doubles, les ortho- ou para- aminophénols, et les bases hétérocycliques, ainsi que les sels d'addition de ces composés avec un acide.

10. Composition selon la revendication 9, **caractérisée par le fait que** les para-phénylènediamines sont choisies parmi les composés de formule (I) suivante : 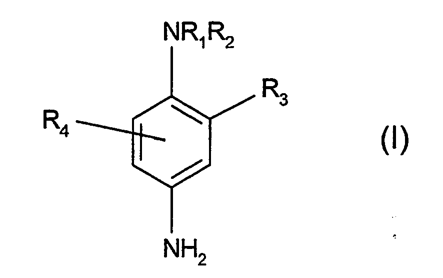 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

11. Composition selon, la revendication 9, **caractérisée par le fait que** les bases doubles sont choisies parmi les composés de structure (II) suivante : 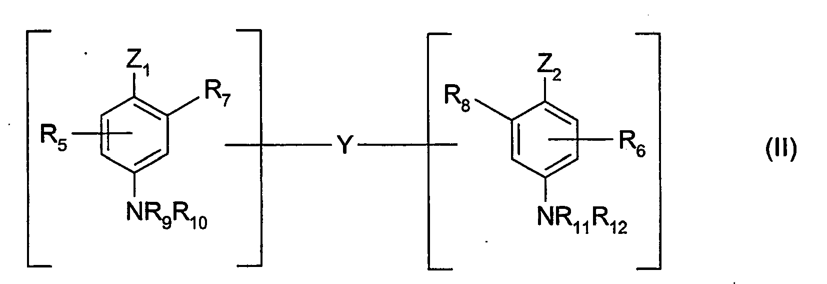 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

12. Composition selon les revendications 10 ou 11, **caractérisée par le fait que** les groupements azotés sont choisis parmi les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

13. Composition selon la revendication 9, **caractérisée par le fait que** les para-aminophénols sont choisis parmi les composés de structure (III) suivante : 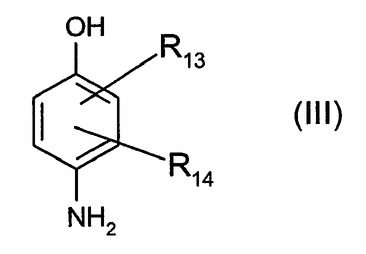 dans laquelle :
R₁₃ représente un atome d'hydrogène, un atome d'halogène, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène, un radical alkyle en C₁₋C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

14. Composition selon la revendication 9, **caractérisée par le fait que** les bases hétérocycliques sont choisies parmi les dérivés pyridiniques, les dérivés pyrimidiniques dont les pyrazolopyrimidines, et les dérivés pyrazoliques.

15. Composition selon les revendications 7 à 14, **caractérisée par le fait que** les bases d'oxydation sont présentes dans des concentrations allant de 0,0005 à 12% et de préférence de 0,005 à 8% en poids par rapport au poids total de la composition.

16. Composition selon la revendication 7, **caractérisée par le fait que** les coupleurs sont choisis parmi les métaphénylènediamines, les métaaminophénols, les métadiphénols, les coupleurs hétérocycliques, et les sels d'addition de ces composés avec un acide.

17. Composition selon les revendications 7 ou 16, **caractérisée par le fait que** les coupleurs sont présents dans des concentrations allant de 0,0001 à 10% et de préférence de 0,005 à 5% en poids par rapport au poids total de la composition.

18. Composition selon l'une des revendications 9 ou 16, **caractérisée par le fait que** les sels d'addition avec un acide des bases d'oxydation et des coupleurs sont choisis parmi les chlorhydrates, les bromhydrates, les sulfates, les tartrates, les lactates et les acétates.

19. Composition selon l'une quelconque des revendications précédentes, **caractérisée par le fait qu'**elle comprend en outre des colorants directs.

20. Composition selon la revendication précédente, **caractérisée par le fait que** la proportion pondérale de colorants directs est comprise entre 0,001 et 20% et de préférence entre 0,01 et 10% du poids total de la composition.

21. Composition selon l'une quelconque des revendications précédentes, **caractérisée par le fait qu'**elle comprend en outre au moins un agent réducteur ou antioxydant, dans des quantités allant de 0,05 à 3% en poids par rapport au poids total de la composition.

22. Composition selon l'une quelconque des revendications précédentes, **caractérisée par le fait qu'**elle comprend au moins un tensioactif choisi parmi les tensioactifs anioniques, cationiques, non ioniques ou amphotères.

23. Composition selon la revendication 22, **caractérisée par le fait qu'**elle comprend au moins un tensioactif anionique de type acide alkyl(C8-C30) éther carboxylique oxyéthyléné ou l'un de ses sels.

24. Composition selon la revendication 22, **caractérisée par le fait qu'**elle comprend au moins un tensioactif non ionique oxyalkyléné ou mono- ou poly- glycérolé.

25. Composition selon l'une quelconque des revendications 22 à 24, **caractérisée par le fait que** les tensioactifs représentent 0,01 à 40% et de préférence de 0,1 à 30% en poids, du poids total de la composition.

26. Composition selon l'une quelconque des revendications précédentes, **caractérisée par le fait qu'**elle comprend au moins un polymère associatif de type non ionique, anionique, cationique ou amphotère dans une proportion allant de 0,01 à 10% en poids par rapport au poids total de la composition.

27. Composition selon l'une quelconque des revendications précédentes, **caractérisée par le fait qu'**elle comprend au moins un polymère cationique ou amphotère dans une proportion allant de 0,01 à 10% en poids par rapport au poids total de la composition.

28. Composition selon l'une quelconque des revendications précédentes, **caractérisée par le fait qu'**elle comprend au moins un alcool gras insaturé dans une proportion en poids allant de 0,5 à 15% par rapport au poids total de la composition.

29. Composition selon l'une des revendications précédentes, **caractérisée en ce qu'**elle comprend, outre l'eau, au moins un solvant choisi parmi les monoalcools ou des diols, linéaire ou ramifiés, de préférence saturés, comprenant 2 à 10 atomes de carbone ; les alcools aromatiques ; les glycols, les éthers de glycol, les alkyl(C1-C4)éthers de diéthylèneglycol ; seuls ou en mélange.

30. Composition selon l'une des revendications précédentes, **caractérisée en ce que** le ou les solvants sont présents, chacun, dans des concentrations comprises entre 0,5 et 20% en poids par rapport au poids total de la composition

31. Composition selon l'une quelconque des revendications précédentes, **caractérisée par le fait qu'**elle se trouve sous la forme d'un liquide.

32. Composition prête à l'emploi pour la teinture d'oxydation des fibres kératiniques en particulier des fibres kératiniques humaines telles que les cheveux, **caractérisée par le fait qu'**elle est susceptible d'être obtenue par mélange d'une composition colorante telle que définie à l'une quelconque des revendications 1 à 31 et d'une composition oxydante comprenant au moins un agent oxydant.

33. Composition selon la revendication 32, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

34. Composition selon la revendication 33, **caractérisée par le fait que** l'agent oxydant est le peroxyde d'hydrogène.

35. Composition selon la revendication 34, **caractérisée par le fait que** l'agent oxydant est une solution d'eau oxygénée dont le titre varie de 1 à 40 volumes.

36. Composition selon l'une quelconque des revendications précédentes, **caractérisée par le fait qu'**elle possède un pH allant de 3 à 12.

37. Procédé de teinture des fibres kératiniques, en particulier pour fibres kératiniques humaines et plus particulièrement des cheveux, **caractérisé par le fait qu'**il consiste à appliquer sur les fibres au moins une composition colorante comprenant, dans un milieu approprié pour la teinture, au moins un colorant d'oxydation et plus de 5 % en poids de la composition d'au moins un amide d'acide gras de colza oxyéthyléné, la couleur étant révélée à pH alcalin, neutre ou acide à l'aide d'une composition oxydante comprenant, dans un milieu approprié pour la teinture, au moins un agent oxydant, qui est mélangée juste au moment de l'emploi à la composition colorante ou qui est appliquée séquentiellement sans rinçage intermédiaire.

38. Procédé selon la revendication 37, **caractérisé par le fait qu'**il consiste à appliquer sur les fibres kératiniques sèches ou humides, la composition prête à l'emploi, réalisée extemporanément au moment de l'emploi à partir des compositions colorante et oxydante, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

39. Dispositif à plusieurs compartiments ou "Kit" pour la teinture des fibres kératiniques humaines et plus particulièrement des cheveux, **caractérisé par le fait qu'**au moins un compartiment renferme une composition colorante comprenant, dans un milieu approprié pour la teinture, au moins un colorant d'oxydation, et plus de 5 % en poids de la composition d'au moins un amide d'acide gras de colza oxyéthyléné et qu'un autre compartiment renferme une composition oxydante comprenant, dans un milieu approprié pour la teinture, un agent oxydant.

## Claims

1. Composition for the oxidation dyeing of keratin fibres, in particular human keratin fibres and more particularly the hair, comprising, in a medium that is suitable for dyeing, at least one oxidation dye, **characterized in that** it comprises more than 5% by weight of the composition of at least one oxyethylenated rapeseed fatty acid amide.

2. Composition according to Claim 1, **characterized in that** the oxyethylenated rapeseed fatty acid amide is chosen from the group formed by those comprising 1 to 20 mol of ethylene oxide.

3. Composition according to Claim 2, **characterized in that** the oxyethylenated rapeseed fatty acid amide is chosen from the group formed by those comprising 1 to 10 mol of ethylene oxide.

4. Composition according to Claim 3, **characterized in that** the oxyethylenated rapeseed fatty acid amide is chosen from the group formed by those comprising 1 to 5 mol of ethylene oxide.

5. Composition according to any one of the preceding claims, **characterized in that** the content of oxyethylenated rapeseed fatty acid amide represents up to 20% by weight of the composition and preferably less than 10% by weight of the composition.

6. Composition according to one of the preceding claims, **characterized in that** the amide content represents at least 5.5% by weight and preferably at least 6% by weight of the composition.

7. Composition according to any one of the preceding claims, **characterized in that** the oxidation dye is chosen from oxidation bases and couplers.

8. Composition according to Claim 7, **characterized in that** it comprises at least one oxidation base.

9. Composition according to Claim 7 or 8,
**characterized in that** the oxidation bases are chosen from ortho- or para-phenylenediamines, double bases, ortho- or para-aminophenols, and heterocyclic bases, and also the addition salts of these compounds with an acid.

10. Composition according to Claim 9, **characterized in that** the para-phenylenediamines are chosen from the compounds of formula (I) below: 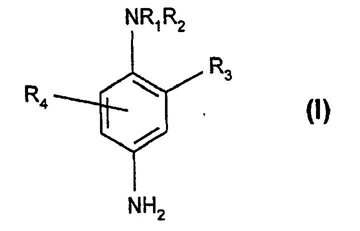 in which:
R₁ represents a hydrogen atom, a C₁-C₄ alkyl radical, a C₁-C₄ monohydroxyalkyl radical, a C₂-C₄ polyhydroxyalkyl radical, a (C₁-C₄) alkoxy(C₁-C₄) alkyl radical or a C₁-C₄ alkyl radical substituted with a nitrogenous, phenyl or 4'-aminophenyl group;
R₂ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
R₁ and R₂ may also form, with the nitrogen atom that bears them, a 5- or 6-membered nitrogenous heterocycle optionally substituted with one or more alkyl, hydroxyl or ureido groups;
R₃ represents a hydrogen atom, a halogen atom, a C₁-C₄ alkyl radical, a sulfo radical, a carboxyl radical, a C₁-C₄ monohydroxyalkyl radical, a C₁-C₄ hydroxyalkoxy radical, an acetylamino (C₁-C₄) alkoxy radical, a mesylamino (C₁-C₄) alkoxy radical or a carbamoylamino (C₁-C₄) alkoxy radical,
R₄ represents a hydrogen or halogen atom or a C₁-C₄ alkyl radical.

11. Composition according to Claim 9, **characterized in that** the double bases are chosen from the compounds of structure (II) below: 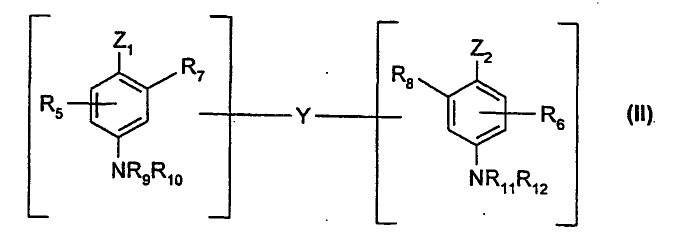 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more heteroatoms such as oxygen, sulfur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

12. Composition according to Claim 10 or 11, **characterized in that** the nitrogenous groups are chosen from amino, mono (C₁-C₄) alkylamino, di (C₁-C₄) alkylamino, tri (C₁-C₄) alkylamino, monohydroxy (C₁-C₄) alkylamino, imidazolinium and ammonium radicals.

13. Composition according to Claim 9, **characterized in that** the para-aminophenols are chosen from the compounds of structure (III) below: 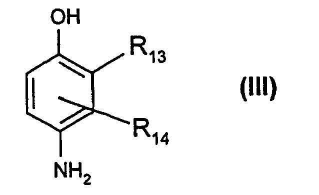 in which:
R₁₃ represents a hydrogen atom, a halogen atom, a C₁-C₄ alkyl radical, a C₁-C₄ monohydroxyalkyl radical, a (C₁-C₄) alkoxy (C₁-C₄) alkyl radical, a C₁-C₄ aminoalkyl radical or a hydroxy (C₁-C₄) alkylamino (C₁-C₄) alkyl radical,
R₁₄ represents a hydrogen atom, a halogen atom, a C₁-C₄ alkyl radical, a C₁-C₄ monohydroxyalkyl radical, a C₂-C₄ polyhydroxyalkyl radical, a C₁-C₄ aminoalkyl radical, a C₁-C₄ cyanoalkyl radical or a (C₁-C₄) alkoxy (C₁-C₄) alkyl radical.

14. Composition according to Claim 9, **characterized in that** the heterocyclic bases are chosen from pyridine derivatives, pyrimidine derivatives including pyrazolopyrimidines, and pyrazole derivatives.

15. Composition according to Claims 7 to 14, **characterized in that** the oxidation bases are present in concentrations ranging from 0.0005% to 12% and preferably from 0.005% to 8% by weight relative to the total weight of the composition.

16. Composition according to Claim 7, **characterized in that** the couplers are chosen from meta-phenylenediamines, meta-aminophenols, meta-diphenols and heterocyclic couplers, and the addition salts of these compounds with an acid.

17. Composition according to Claim 7 or 16, **characterized in that** the couplers are present in concentrations ranging from 0.0001% to 10% and preferably from 0.005% to 5% by weight relative to the total weight of the composition.

18. Composition according to either of Claims 9 and 16, **characterized in that** the addition salts with an acid of the oxidation bases and couplers are chosen from the hydrochlorides, hydrobromides, sulfates, tartrates, lactates and acetates.

19. Composition according to any one of the preceding claims, **characterized in that** it also comprises direct dyes.

20. Composition according to the preceding claim, **characterized in that** the weight proportion of direct dyes is between 0.001% and 20% and preferably between 0.01% and 10% relative to the total weight of the composition.

21. Composition according to any one of the preceding claims, **characterized in that** it also comprises at least one reducing agent or antioxidant, in amounts ranging from 0.05% to 3% by weight relative to the total weight of the composition.

22. Composition according to any one of the preceding claims, **characterized in that** it comprises at least one surfactant chosen from anionic, cationic, nonionic and amphoteric surfactants.

23. Composition according to Claim 22, **characterized in that** it comprises at least one anionic surfactant of oxyethylenated (C₈-C₃₀)alkyl ether carboxylic acid type or a salt thereof.

24. Composition according to Claim 22, **characterized in that** it comprises at least one oxyalkylenated or monoglycerolated or polyglycerolated nonionic surfactant.

25. Composition according to any one of Claims 22 to 24, **characterized in that** the surfactants represent 0.01% to 40% and preferably from 0.1% to 30% by weight relative to the total weight of the composition.

26. Composition according to any one of the preceding claims, **characterized in that** it comprises at least one associative polymer of the nonionic, anionic, cationic or amphoteric type in a proportion ranging from 0.01% to 10% by weight relative to the total weight of the composition.

27. Composition according to any one of the preceding claims, **characterized in that** it comprises at least one cationic or amphoteric polymer in a proportion ranging from 0.01% to 10% by weight relative to the total weight of the composition.

28. Composition according to any one of the preceding claims, **characterized in that** it comprises at least one unsaturated fatty alcohol in a weight proportion ranging from 0.5% to 15% relative to the total weight of the composition.

29. Composition according to one of the preceding claims, **characterized in that** it comprises, besides water, at least one solvent chosen from linear or branched, preferably saturated, monoalcohols or diols containing 2 to 10 carbon atoms; aromatic alcohols; glycols, glycol ethers or diethylene glycol (C₁-C₄)alkyl ethers; alone or as a mixture.

30. Composition according to one of the preceding claims, **characterized in that** the solvent(s) is (are) each present in concentrations of between 0.5% and 20% by weight relative to the total weight of the composition.

31. Composition according to any one of the preceding claims, **characterized in that** it is in the form of a liquid.

32. Ready-to-use composition for the oxidation dyeing of keratin fibres, in particular human keratin fibres such as the hair, **characterized in that** it may be obtained by mixing a dye composition as defined in any one of Claims 1 to 31 and an oxidizing composition comprising at least one oxidizing agent.

33. Composition according to Claim 32, **characterized in that** the oxidizing agent is chosen from hydrogen peroxide, urea peroxide, alkali metal bromates or ferricyanides, persalts, redox enzymes such as laccases, peroxidases and 2-electron oxidoreductases, where appropriate in the presence of the respective donor or cofactor thereof.

34. Composition according to Claim 33, **characterized in that** the oxidizing agent is hydrogen peroxide.

35. Composition according to Claim 34, **characterized in that** the oxidizing agent is an aqueous hydrogen peroxide solution with a titre ranging from 1 to 40 volumes.

36. Composition according to any one of the preceding claims, **characterized in that** it has a pH ranging from 3 to 12.

37. Process for dyeing keratin fibres, in particular human keratin fibres and more particularly the hair, **characterized in that** it consists in applying to the fibres at least one dye composition comprising, in a medium that is suitable for dyeing, at least one oxidation dye and more than 5% by weight of the composition of at least one oxyethylenated rapeseed fatty acid amide, the colour being developed at alkaline, neutral or acidic pH using an oxidizing composition comprising, in a medium that is suitable for dyeing, at least one oxidizing agent, which is mixed just at the time of use with the dye composition or which is applied sequentially without intermediate rinsing.

38. Process according to Claim 37, **characterized in that** it consists in applying to the wet or dry keratin fibres the ready-to-use composition prepared extemporaneously at the time of use from the dye composition and the oxidizing composition, leaving the composition to act for a leave-in time ranging from 1 to 60 minutes approximately and preferably from 10 to 45 minutes, rinsing the fibres, then optionally washing them with shampoo, and then rinsing them again and drying them.

39. Multi-compartment device or "kit" for the dyeing of human keratin fibres and more particularly the hair, **characterized in that** at least one compartment contains a dye composition comprising, in a medium that is suitable for dyeing, at least one oxidation dye and more than 5% by weight of the composition of at least one oxyethylenated rapeseed fatty acid amide, and another compartment contains an oxidizing composition comprising, in a medium that is suitable for dyeing, an oxidizing agent.

## Patentansprüche

1. Zusammensetzung zum oxidativen Färben von Keratinfasern und insbesondere menschlichen Keratinfasern, besonders Haaren, die in einem zum Färben geeigneten Medium mindestens einen Oxidationsfarbstoff enthält, **dadurch gekennzeichnet, dass** sie ferner mindestens ein ethoxyliertes Rapsfettsäureamid in einer Menge von mehr als 5 Gew.-% enthält.

2. Zusammensetzung nach Anspruch 1, **dadurch gekennzeichnet, dass** das ethoxylierte Rapsfettsäureamid unter den Verbindungen mit 1 bis 20 Mol Ethylenoxid ausgewählt ist.

3. Zusammensetzung nach Anspruch 2, **dadurch gekennzeichnet, dass** das ethoxylierte Rapsfettsäureamid unter den Verbindungen mit 1 bis 10 Mol Ethylenoxid ausgewählt ist.

4. Zusammensetzung nach Anspruch 3, **dadurch gekennzeichnet, dass** das ethoxylierte Rapsfettsäureamid unter den Verbindungen mit 1 bis 5 Mol Ethylenoxid ausgewählt ist.

5. Zusammensetzung nach einem der vorhergehenden Ansprüche, **dadurch gekennzeichnet, dass** der Gehalt des ethoxylierten Rapsfettsäureamids bis zu 20 Gew.-% der Zusammensetzung ausmacht und vorzugsweise unter 10 Gew.-% der Zusammensetzung liegt.

6. Zusammensetzung nach einem der vorhergehenden Ansprüche, **dadurch gekennzeichnet, dass** der Gehalt des ethoxylierten Rapsfettsäureamids mindestens 5,5 Gew.-% der Zusammensetzung und vorzugsweise mindestens 6 Gew.-% der Zusammensetzung ausmacht.

7. Zusammensetzung nach einem der vorhergehenden Ansprüche, **dadurch gekennzeichnet, dass** der Oxidationsfarbstoff unter den Oxidationsbasen und Kupplern ausgewählt ist.

8. Zusammensetzung nach Anspruch 7, **dadurch gekennzeichnet, dass** sie mindestens eine Oxidationsbase enthält.

9. Zusammensetzung nach den Ansprüchen 7 oder 8, **dadurch gekennzeichnet, dass** die Oxidationsbasen unter den o- oder *p*-Phenylendiaminen, Doppelbasen, o- oder p-Aminophenolen und den heterocyclischen Basen sowie den Additionssalzen dieser Verbindungen mit einer Säure ausgewählt sind.

10. Zusammensetzung nach Anspruch 9, **dadurch gekennzeichnet, dass** die p-Phenylendiamine unter den Verbindungen der folgenden Formel (I) ausgewählt sind: 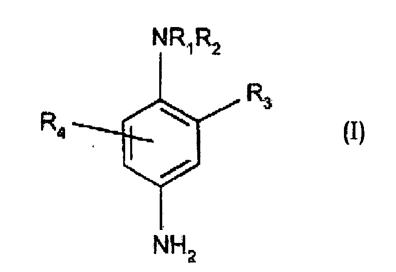 worin bedeuten:
R₁ ein Wasserstoffatom, C₁₋₄-Alkyl, C₁₋₄-Monohydroxyalkyl, C₂₋₄₋Polyhydroxyalkyl, Alkoxy(C₁₋₄)alkyl(C₁₋₄), eine mit einer Stickstoff-haltigen Gruppe substituierte C₁₋₄-Alkylgruppe, Phenyl oder 4'-Aminophenyl;
R₂ ein Wasserstoffatom, C₁₋₄-Alkyl, C₁₋₄-Monohydroxyalkyl, C₂₋₄₋Polyhydroxyalkyl, Alkoxy(C₁₋₄)alkyl(C₁₋₄) oder eine mit einer Stickstoff-haltigen Gruppe substituierte C₁₋₄-Alkylgruppe, wobei R₁ und R₂ auch mit dem Stickstoffatom, von dem sie getragen werden, einen 5- oder 6-gliedrigen Stickstoffheterocyclus bilden können, der gegebenenfalls mit einer oder mehreren Gruppen Alkyl, Hydroxy oder Ureido substituiert ist;
R₃ ein Wasserstoffatom, ein Halogenatom, C₁₋₄-Alkyl, Sulfo, Carboxy, C₁₋₄-Monohydroxalkyl, C₁₋₄-Hydroxyalkoxy, C₁₋₄₋Acetylaminoalkoxy, C₁₋₄-Mesylaminoalkoxy oder C₁₋₄₋Carbamoylaminoalkoxy,
R₄ ein Wasserstoffatom, ein Halogenatom oder C₁₋₄-Alkyl.

11. Zusammensetzung nach Anspruch 9, **dadurch gekennzeichnet, dass** die Doppelbasen unter den Verbindungen der folgenden Struktur (II) ausgewählt sind: 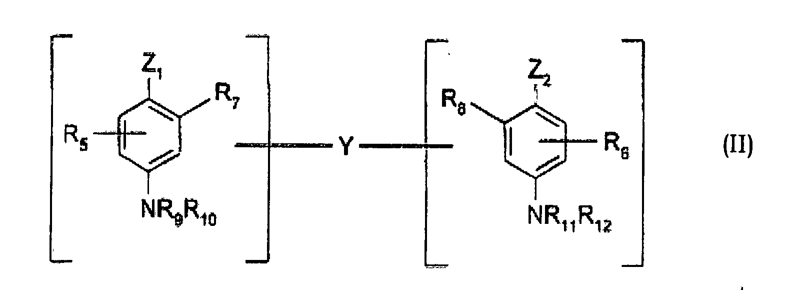 worin bedeuten:
- die Gruppen Z₁ und Z₂, die gleich oder verschieden sind, die Hydroxygruppe oder die Gruppe -NH₂, die mit einer C₁₋₄-Alkylgruppe oder einer Verbindungsgruppe Y substituiert sein kann;
- die Verbindungsgruppe Y eine geradkettige oder verzweigte Alkylenkette mit 1 bis 14 Kohlenstoffatomen, die durch eine oder mehrere Stickstoff-haltige Gruppen und/oder ein oder mehrere Heteroatome, wie Sauerstoff, Schwefel oder Stickstoff, unterbrochen oder abgeschlossen sein kann und gegebenenfalls mit einer oder mehreren Hydroxygruppen oder C₁₋₆-Alkoxygruppen substituiert ist;
- die Gruppen R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y;
- die Gruppen R₇, R₈, R₉, R₁₀, R₁₁ und R₁₂, die gleich oder verschieden sind, ein Wasserstoffatom, eine Verbindungsgruppe Y oder eine C₁₋₄-Alkylgruppe;
mit der Maßgabe, dass die Verbindungen der Formel (II) nur eine Verbindungsgruppe Y pro Molekül enthalten.

12. Zusammensetzung nach den Ansprüchen 10 oder 11, **dadurch gekennzeichnet, dass** die Stickstoff-haltigen Gruppen unter den Gruppen Amino, Monoalkyl(C₁₋₄)amino, Dialkyl(C₁₋₄)amino, Trialkyl(C₁₋₄)amino, Monohydroxyalkyl(C₁₋₄)amino, Imidazolinium und Ammonium ausgewählt sind.

13. Zusammensetzung nach Anspruch 9, **dadurch gekennzeichnet, dass** die p-Aminophenole unter den Verbindungen der folgenden Struktur (III) ausgewählt sind: 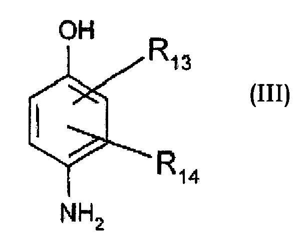 worin bedeuten:
R₁₃ ein Wasserstoffatom, ein Halogenatom, C₁₋₄-Alkyl, C₁₋₄₋Monohydroxyalkyl, Alkoxy(C₁₋₄)alkyl(C₁₋₄), C₁₋₄-Aminoalkyl oder Hydroxyalkyl(C₁₋₄)aminoalkyl(C₁₋₄),
R₁₄ ein Wasserstoffatom, ein Halogenatom, C₁₋₄-Alkyl, C₁₋₄₋Monohydroxyalkyl, C₂₋₄-Polyhydroxyalkyl, C₁₋₄-Aminoalkyl, C₁₋₄₋Cyanoalkyl oder Alkoxy(C₁₋₄)alkyl(C₁₋₄).

14. Zusammensetzung nach Anspruch 9, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

15. Zusammensetzung nach den Ansprüchen 7 bis 14, **dadurch gekennzeichnet, dass** die Oxidationsbasen in Konzentrationen von 0,0005 bis 12 Gew.-% und vorzugsweise 0,005 bis 8 Gew.-%, bezogen auf das Gesamtgewicht der Zusammensetzung, enthalten sind.

16. Zusammensetzung nach Anspruch 7, **dadurch gekennzeichnet, dass** die Kuppler unter den *m*-Phenylendiaminen, m-Aminophenolen, *m*-Dihydroxybenzolen, heterocyclischen Kupplern und den Additionssalzen dieser Verbindungen mit einer Säure ausgewählt sind.

17. Zusammensetzung nach den Ansprüchen 7 oder 16, **dadurch gekennzeichnet, dass** die Kuppler in Konzentrationen von 0,0001 bis 10 Gew.-% und vorzugsweise 0,005 bis 5 Gew.-%, bezogen auf das Gesamtgewicht der Zusammensetzung, enthalten sind.

18. Zusammensetzung nach den Ansprüchen 9 oder 16, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

19. Zusammensetzung nach einem der vorhergehenden Ansprüche, **dadurch gekennzeichnet, dass** sie ferner Direktfarbstoffe enthält.

20. Zusammensetzung nach dem vorhergehenden Anspruch, **dadurch gekennzeichnet, dass** der Mengenanteil der Direktfarbstoffe im Bereich von 0,001 bis 20 % und vorzugsweise 0,01 bis 10 % des Gesamtgewichts der Zusammensetzung liegt.

21. Zusammensetzung nach einem der vorhergehenden Ansprüche, **dadurch gekennzeichnet, dass** sie ferner mindestens ein Reduktionsmittel oder Antioxidationsmittel in Mengenanteilen von 0,05 bis 3 Gew.-%, bezogen auf das Gesamtgewicht der Zusammensetzung, enthält.

22. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren grenzflächenaktiven Stoffen ausgewählt ist.

23. Zusammensetzung nach Anspruch 22, **dadurch gekennzeichnet, dass** sie mindestens einen anionischen grenzflächenaktiven Stoff von Typ der ethoxylierten Alkyl(C8-C30)ethercarbonsäuren oder ihrer Salze enthält.

24. Zusammensetzung nach Anspruch 22, **dadurch gekennzeichnet, dass** sie mindestens einen alkoxylierten oder ein- oder mehrfach mit Glycerin veretherten, nichtionischen grenzflächenaktiven Stoff enthält.

25. Zusammensetzung nach einem der Ansprüche 22 bis 24, **dadurch gekennzeichnet, dass** die grenzflächenaktiven Stoffe 0,01 bis 40 % und vorzugsweise 0,1 bis 30 Gew.-% des Gesamtgewichts der Zusammensetzung ausmachen.

26. Zusammensetzung nach einem der vorhergehenden Ansprüche, **dadurch gekennzeichnet, dass** sie mindestens ein assoziatives Polymer vom nichtionischen, anionischen, kationischen oder amphoteren Typ in einer Menge von 0,01 bis 10 Gew.-%, bezogen auf das Gesamtgewicht der Zusammensetzung, enthält.

27. Zusammensetzung nach einem der vorhergehenden Ansprüche, **dadurch gekennzeichnet, dass** sie mindestens ein kationisches Polymer oder ein amphoteres Polymer in einer Menge von 0,01 bis 10 Gew.-%, bezogen auf das Gesamtgewicht der Zusammensetzung, enthält.

28. Zusammensetzung nach einem der vorhergehenden Ansprüche, **dadurch gekennzeichnet, dass** sie mindestens einen ungesättigten Fettalkohol in Mengenanteilen von 0,5 bis 15 %, bezogen auf das Gesamtgewicht der Zusammensetzung, enthält.

29. Zusammensetzung nach einem der vorhergehenden Ansprüche, **dadurch gekennzeichnet, dass** sie neben Wasser mindestens ein Lösungsmittel enthält, das unter den geradkettigen oder verzweigten, vorzugsweise gesättigten Monoalkoholen oder Diolen mit 2 bis 10 Kohlenstoffatomen; aromatischen Alkoholen; Glycolen; Glycolethern, Alkyl(C1-C4)ethern von Diethylenglycol; oder deren Gemischen ausgewählt ist.

30. Zusammensetzung nach einem der vorhergehenden Ansprüche, **dadurch gekennzeichnet, dass** das oder die Lösungsmittel jeweils in Konzentrationen von 0,5 bis 20 Gew.-%, bezogen auf das Gesamtgewicht der Zusammensetzung, enthalten sind.

31. Zusammensetzung nach einem der vorhergehenden Ansprüche, **dadurch gekennzeichnet, dass** sie als Flüssigkeit vorliegt.

32. Gebrauchsfertige Zusammensetzung zum oxidativen Färben von Keratinfasern und insbesondere menschlichen Keratinfasern, wie Haaren, **dadurch gekennzeichnet, dass** sie durch Mischen einer Farbmittelzusammensetzung nach einem der Ansprüche 1 bis 31 und einer oxidierenden Zusammensetzung, die mindestens ein Oxidationsmittel enthält, erhältlich ist.

33. Zusammensetzung nach Anspruch 32, **dadurch gekennzeichnet, dass** das Oxidationsmittel unter Wasserstoffperoxid, Harnstoff peroxid, Alkalimetallbromaten, Alkalimetallferricyaniden, Salzen von Persäuren, Redoxenzymen, wie Laccasen, Peroxidasen und Oxidoreduktasen (2 Elektronen) gegebenenfalls in Gegenwart ihres Donors oder jeweiligen Cofaktors ausgewählt ist.

34. Zusammensetzung nach Anspruch 33, **dadurch gekennzeichnet, dass** es sich bei dem Oxidationsmittel um Wasserstoffperoxid handelt.

35. Zusammensetzung nach Anspruch 34, **dadurch gekennzeichnet, dass** das Oxidationsmittel eine Wasserstoffperoxidlösung ist, deren Titer im Bereich von 1 bis 40 Volumina liegt.

36. Zusammensetzung nach einem der vorhergehenden Ansprüche, **dadurch gekennzeichnet, dass** sie einen pH-Wert von 3 bis 12 aufweist.

37. Verfahren zum Färben von Keratinfasern und insbesondere menschlichen Keratinfasern, besonders zum Färben der Haare, **dadurch gekennzeichnet, dass** es darin besteht, mindestens eine Farbmittelzusammensetzung, die in einem zum Färben geeigneten Medium mindestens einen Oxidationsfarbstoff und mehr als 5 Gew.-% mindestens eines ethoxylierten Rapsfettsäureamids enthält, auf die Fasern aufzutragen, wobei die Farbe bei einem alkalischen, neutralen oder sauren pH-Wert mit einer oxidierenden Zusammensetzung gebildet wird, die in einem zum Färben geeigneten Medium mindestens ein Oxidationsmittel enthält und die kurz vor der Anwendung mit der Farbmittelzusammensetzung vermischt wird oder die ohne zwischenzeitliches Spülen getrennt davon anschließend aufgetragen wird.

38. Verfahren nach Anspruch 37, **dadurch gekennzeichnet, dass** es darin besteht, auf die trockenen oder feuchten Keratinfasern die gebrauchsfertige Zusammensetzung aufzutragen, die bedarfsgemäß bei der Anwendung aus der Farbmittelzusammensetzung und der oxidierenden Zusammensetzung gebildet wird, sie während einer Zeitspanne von etwa 1 bis 60 Minuten und vorzugsweise 10 bis 45 Minuten einwirken zu lassen, die Fasern zu spülen, gegebenenfalls mit Haarwaschmittel zu waschen, nochmals zu spülen und zu trocknen.

39. Vorrichtung mit mehreren Abteilungen oder 'Kit' zum Färben von menschlichen Keratinfasern und insbesondere Haaren, **dadurch gekennzeichnet, dass** mindestens eine Abteilung eine Farbmittelzusammensetzung enthält, die in einem zum Färben geeigneten Medium mindestens einen Oxidationsfarbstoff und mehr als 5 Gew.-% mindestens eines ethoxylierten Rapsfettsäureamids enthält, und eine weitere Abteilung eine oxidierende Zusammensetzung enthält, die in einem zum Färben geeigneten Medium mindestens ein Oxidationsmittel enthält.
